# EUROPÄISCHE PATENTANMELDUNG

(11) **EP 1 782 760 A1**
(43) Veröffentlichungstag der Anmeldung: **09.05.2007**
(21) Anmeldenummer: 05024117.3
(22) Anmeldetag: 04.11.2005
(51) Int. Cl.: A61F 2/04, A61B 17/12, A61L 31/14, A61B 17/08

(54) **Implantat, vorzugsweise zur Behandlung der gastroösophagealen Refluxkrankheit**

(71) Anmelder: Haugen, Haavard, 0264 Oslo (NO); Will, Julia, Dr., 80807 München (DE); Feussner, Hubertus, Prof. Dr., 81671 München (DE)
(72) Erfinder: Haugen, Haavard, 0264 Oslo (NO); Will, Julia, Dr., 80807 München (DE); Feussner, Hubertus, Prof. Dr., 81671 München (DE)
(74) Vertreter: Herrmann, Uwe

(57) **Zusammenfassung**

Die Erfindung betrifft ein Implantat, vorzugsweise ein Implantat zur Behandlung der gastroösophagealen Reflux-Krankheit, mit einem Kontaktbereich, mit dem das lmplantat im implantierten Zustand mit Körpergewebe in Verbindung steht, wobei der Kontaktbereich wenigstens bereichsweise eine poröse Struktur aufweist.

## Beschreibung

Die Erfindung betrifft ein Implantat, vorzugsweise ein Implantat zur Behandlung der gastroösophagealen Refluxkrankheit.

Die Ursache für die Refluxkrankheit, die zu Sodbrennen führen kann, ist eine unzureichende Funktion des Schließmuskels zwischen Speiseröhre und Magen, die zu einem Rückfluß der Magensäure in die Speisröhre führen kann. Für diese als Reflux bekannte Funktionsstörung gibt es unterschiedliche Gründe. In Betracht kommt eine Funktionsschwäche des Speiseröhren-Schließmuskels, ein erniedrigter Ruhetonus (Spannungszustand der quergestreiften Muskulatur im Zustand körperlicher Ruhe) und/oder Störungen der Peristaltik.

Ein geringfügiger Rückfluß von Mageninhalt in die Speiseröhre, beispielsweise nach der Einnahme fetter Speisen, ist normal. Wenn jedoch Sodbrennen und Schmerzen regelmäßig auftreten, ist abzuklären, ob als Ursache der Beschwerden eine Refluxösophagitis ausgemacht werden kann, d. h. ob eine Entzündung des unteren Abschnittes der Speiseröhre vorliegt, die durch die ständige Reizwirkung der in die Speiseröhre eingedrungenen Magensäure entsteht.

Etwa 5 % bis 10 % der Bevölkerung leiden unter der gastroösophagealen Reflux-krankheit. Diese führt gegebenenfalls zu einer dauerhaften Verringerung der Lebensqualität der Betroffenen und kann auf lange Sicht auch zu einer Krebsentwicklung aufgrund Refluxösophagitis der betroffenen Bereiche führen.

Es sind zahlreiche Möglichkeiten bekannt, eine Refluxkrankheit zu behandeln. Neben der Änderung des Lebensstils kommen medikamentöse, operative oder neuerdings auch endoskopische Verfahren in Betracht.

Es ist bekannt, daß Protonenpumpenblocker die Salzsäureproduktion der Magenschleimhaut reduzieren. Auf diese Weise läßt sich die Aggressivität des Reflorats, nicht jedoch das Refluxvolumen reduzieren. Die Anwendung von Protonenpumpenblockern stellt für die Mehrzahl aller Patienten eine erfolgreiche medikamentöse Therapie dar. Je nach Schweregrad der Refluxösophagitis werden Antazida, z. B. Magnesium- oder Aluminiumhydroxid (bei gelegentlichen Beschwerden), H₂-Rezeptorenblocker, z. B. Ranitidin (bei intensiven Beschwerden) oder Protonenpumpenhemmer, z. B. Omeprazol (bei länger als sechs Wochen anhaltenden Beschwerden) eingesetzt. Die beiden letztgenannten Medikamente blockieren das HCl-bildende Enzym Histamin (Protonenpumpe) in den säurebildenden Zellen des Magens, den sogenannten Belegzellen. Die vorgenannten Protonenpumpenhemmer sind derzeit immer noch die am häufigsten angewandten und vielversprechendsten Medikamente gegen Refluxösophagitis. Es ist bekannt, daß eine langfristige Erfolgsrate von 80 % bis 90 % erreicht werden kann. Die Anwendung von Protonenpumpenhemmern erfordert eine lebenslange Behandlung. Problematisch kann die Bereitschaft der Betroffenen sein, sich einer permanenten medikamentösen Therapie zu unterziehen.

Alternativ oder zusätzlich zu der genannten medikamentösen Behandlung kommen gegebenenfalls endoluminale Behandlungsverfahren in Betracht, die derzeit nur unter Studienbedingungen untersucht werden. Die Indikation derartiger Verfahren ist auf relativ seltene Sonderfälle beschränkt.

Ein Beispiel für ein endoluminales Behandlungsverfahren basiert auf dem "Bard Interventional Endoscopic Suturing System". Dieses System bzw. die damit durchgeführte Methode basiert darauf, das Gewebe vom Magen mit einem speziellen Gerät angesaugt und mittels einer Art "Nähmaschine" angenäht wird. Ein weiteres Beispiel ist der Einsatz kleiner metallischen Implantate (Curon's Stretta), die in die Muskulatur um den Magensphingter eingebracht werden. Bei diesem Verfahren wird die kontrollierte Applikation von Radiofrequenz-Energie im Bereich des unteren Ösophagussphingters angewandt, wodurch eine erhebliche Besserung der Refluxsymptome und eine Verkürzung der Säureexpositionsdauer erreicht werden konnte. Die Implantate dienen als Antennen, um Hitze mittels Hochfrequenz einzubringen. Die Idee dieses Verfahrens basiert darauf, daß der Durchmesser der Speiseröhre sich aufgrund der durch die Hitze induzierten Narben verringert.

Eine weitere Möglichkeit, auf operativem Wege Refluxösophagitis zu behandeln, ist die sogenannte Angelchik Antireflux-Prothese. Diese besteht aus einem silikongefüllten Elastomerschlauch von ca. 20 mm Durchmesser, der mit Hilfe eines Silikonbandes zu einem Ring geschlossen um die Speiseröhre gelegt wird. Der Ring verengt die Speiseröhre zur Unterstützung des Speisröhrenschließmuskels und entlastet diesen somit.

Eine weitere Methode ist der Einsatz eines Vicryl-Bandes, das um den unteren Teil der Speiseröhre gelegt wird.

Weiterhin ist es bekannt, eine endoskopische Injektion eines biologisch nicht abbaubaren Polymers in die Ösophaguswand vorzunehmen, die durch Bildung einer fibrösen Abkapselung eine Anhebung des Verschlußdruckes des unteren Ösophagus und damit eine Linderung der Symptome bewirkt. Bei den vorzugsweise quellfähigen Stoffen handelt es sich beispielsweise um bovines Collagen, Polymethamethyacrylate und Polytetrafluoroethylenepaste. Bekannt ist eine Methode, wobei Ethylen Vinyl Alkohol mit Tantal-Partikeln submuscosal injiziert werden. Auch diese Methode bewirkt eine Verringerung des Durchmessers des Ösophagus.

Die oben genannte medikamentöse Behandlung lindert die Beschwerden, weist jedoch den Nachteil auf, daß die zugrunde liegende Krankheit nicht geheilt wird. Der Reflux von Magensäure kann weiterhin die Schleimhaut der Speiseröhre schädigen, was die oben genannten Nachteile mit sich bringen kann.

Auch die genannten Methoden haben trotz ihres erfolgreichen Einsatzes alle ernsthafte Komplikationen. Neben- oder Folgewirkungen können das Lockern der Nähte mit einem erneuten Rückfall sein, Magenfisteln, eine Stenose, das Verrutschen des Implantates bei der Angelchik-Methode, die Perforation des Magens oder sogar der Austritt des Implantates durch das Zwerchfell.

Das Spektrum der operativen Behandlungsmethoden ist außerordentlich breit. Üblicherweise werden heute verschiedene Formen der Manschettenbildung um den ösophagokardialen Übergang bevorzugt. Zumindest in größeren Zentren werden diese Eingriffe minimal-invasiv (laparoskopisch) durchgeführt. Die chirurgische Antirefluxbehandlung wirkt prompt und dauerhaft. Sie ist in einem gewissen Prozentsatz jedoch mit intraoperativen und insbesondere mit postoperativen Komplikationen und Nebenwirkungen behaftet. Die oben genannte Angelchik-Prothese ist aufgrund der hohen Rate an fremdkörperbedingten Nebenwirkungen (Migration, Perforation etc.) heute obsolet. Alternativ wurde die Implantation von voll oder partiell resorbierbaren Bändern bzw. Schals untersucht. Zu einer klinischen Anwendung kann es aufgrund des nur temporären Effektes (bei vollständig resorbierbaren Schals) bzw. der üblichen Fremdkörperproblematik (partiell resorbierbare Schals) nicht.

Zusammenfassend ergibt sich somit folgendes:

In der Mehrzahl aller Fälle ist die medikamentöse Therapie mit Protonenpumpenblockern ausreichend, sofern sich seitens der Patienten die Bereitschaft zu einer lang andauernden medikamentösen Therapie findet. Bei einer kleineren Anzahl von Patienten ist nach wie vor die chirurgische Behandlung aufgrund von Volumenreflux, Mediakamentenunverträglichkeit, fehlender Compliance etc. erforderlich. Die oben genannten interventionellen (endoluminalen) Verfahren spielen in absehbarer Zeit noch keine Rolle. Die chirurgische Antirefluxtherapie in Form der sogenannten Manschettenbildung (Fundoplicatio) wirkt zuverlässig, ist jedoch technisch relativ anspruchsvoll und darüber hinaus mit einer Komplikations- bzw. Nebenwirkungsrate in Höhe von etwa 10 % bis 15 % der Fälle belastet.

Es ist die Aufgabe der vorliegenden Erfindung, ein Implantat der eingangs genannten Art dahingehend weiterzubilden, daß eine wenig invasive, technisch einfache chirurgische Behandlungsoption eröffnet wird, wobei die Komplikationsrate insbesondere durch das Verrutschen des Implantates gegenüber vorbekannten Lösungen verringert ist.

Diese Aufgabe wird durch ein Implantat mit den Merkmalen des Anspruchs 1 gelöst. Die Erfindung betrifft ein Implantat, vorzugsweise ein Implantat zur Behandlung der gastroösophagealen Reflux-Krankheit, mit einem Kontaktbereich, mit dem das Implantat im implantiertem Zustand mit Körpergewebe in Verbindung steht, wobei der Kontaktbereich wenigstens bereichsweise eine poröse Struktur aufweist. Die Porosität des Kontaktbereichs bringt den erheblichen Vorteil mit sich, daß Fibroblasten in die poröse Struktur hineinwachsen können, so daß aufgrund der Vaskularisation ein Wandern des Implantates wirksam verhindert wird. Wird das Implantat zur Behandlung der gastroösophagealen Reflux-Krankheit eingesetzt, ermöglicht die poröse Struktur des Kontaktbereiches ein Einwachsen des Speiseröhrengewebes in den Kontaktbereich. Das Risiko des Verrutschens des Implantates, wie es bei der Angelchik Antireflux-Prothese beobachtet wurde, ist somit wesentlich reduziert. Besonders vorteilhaft ist es, wenn sich die poröse Struktur nicht nur in dem Kontaktbereich erstreckt, in dem das Implantat am Gewebe anliegt, sondern darüber hinaus auch dem Kontaktbereich benachbarte Bereiche des Implantates eine poröse Struktur aufweisen.

Besonders vorteilhaft ist es, wenn die Poren der porösen Struktur miteinander in Verbindung stehen, d. h. interkonnektiv ausgeführt sind. Besonders vorteilhafte Porengrößen liegen im Bereich zwischen 100 und 300 µm. Die Größe der Verbindungen zwischen diesen Poren, das heißt die Größe der interkonnektiven Poren liegt vorzugsweise im Bereich zwischen 10 und 30 µm. Die Porosität liegt vorzugsweise bei mindestens 50 % und besonders bevorzugt bei mindestens 65 %. Selbstverständlich sind auch davon abweichende Porengrößen bzw. Porositäten denkbar.

Die angegebenen Porositäten bzw. Porengrößen sind im Hinblick auf das Einwachsen von Fibroblasten in das Implantat besonders vorteilhaft.

Besonders vorteilhaft ist eine offenporige Struktur der porösen Bereiche.

In weiterer Ausgestaltung der Erfindung ist vorgesehen, daß die nicht den Kontaktbereich des Implantates bildende Oberfläche glatt ausgeführt ist. Auf diese Weise kann ein unerwünschtes Anwachsen von benachbartem Gewebe oder anderen Organen an das Implantat verhindert werden.

Besonders vorteilhaft ist es, wenn das Implantat einen Aufnahmebereich zur wenigstens teilweise Aufnahme von Körpergewebe, insbesondere eines Abschnittes der Speiseröhre aufweist. Der Aufnahmebereich kann beispielsweise im wesentlichen kreisförmig ausgeführt sein, was bei der Behandlung der gastroösophagealen Reflux-Krankheit denkbar ist. Um eine besonders gute Anpaßbarkeit des Implantates an unterschiedliche Gegebenheiten, beispielsweise unterschiedliche Durchmesser der Speiseröhre, zu gewährleisten, kann vorgesehen sein, daß die Größe des Aufnahmebereichs veränderbar ist. Auf diese Weise läßt sich der Vorteil realisieren, daß die Bereitstellung eines Implantates für eine Vielzahl von Patienten/Anwendungen ausreicht.

Das Implantat kann einen im wesentlichen ringförmigen Abschnitt aufweisen, der den Aufnahmebereich zumindest teilweise umgibt. Vorzugsweise ist dessen Umfang veränderbar.

In weiterer Ausgestaltung der Erfindung ist vorgesehen, daß das Implantat einen steckerförmigen Abschnitt und eine Steckeraufnahme aufweist, in die der steckerförmige Abschnitt einführbar ist. Dabei kann vorgesehen sein, daß der steckerförmige Abschnitt über ein oder mehrere Arretierungsmittel verfügt, mittels derer der steckerförmige Abschnitt in der Steckeraufnahme arretierbar ist. Sind mehrere Arretierungsmittel vorgesehen, läßt sich der Vorteil erreichen, daß unterschiedliche Einsteckpositionen und somit unterschiedliche Größen des Aufnahmebereiches des Implantates realisierbar sind.

In einer bevorzugten Ausgestaltung der Erfindung weist das Implantat einen den Aufnahmebereich zumindest teilweise umgebenden Abschnitt auf, an dessen einem Endbereich sich der steckerförmige Abschnitt und an dessen anderem Endbereich die Steckeraufnahme angeordnet ist.

In einer weiteren Ausgestaltung der Erfindung ist vorgesehen, daß das Implantat einen die poröse Struktur aufweisenden Kern sowie eine Umhüllung aufweist, die den Kern außer im Kontaktbereich des Implantates umgibt. Um ein unerwünschtes Anwachsen von Gewebe oder Organen zu verhindern, weist die Umhüllung vorzugsweise die genannte glatte Oberfläche auf.

Weiterhin kann vorgesehen sein, daß die poröse Struktur ein oder mehrere Polymere enthält oder aus einem oder mehreren Polymeren besteht. In Betracht kommt beispielsweise die Verwendung von Polyurethan. In bevorzugter Ausgestaltung der Erfindung besteht das Implantat aus nur einem Polymer, vorzugsweise aus Polyurethan.

Weitere Einzelheiten und Vorteile der Erfindung werden anhand eines in der Zeichnung dargestellten Ausführungsbeispiels näher erläutert. Es zeigen:
- Fig. 1:: unterschiedliche perspektivische Darstellungen des Implantates gemäß der Erfindung,
- Fig. 2, 3:: unterschiedliche Ansichten/Schnittansichten des Implantates gemäß der Erfindung,
- Fig. 4:: ein Verfahrensschema zur Herstellung der porösen Struktur des Implantates und
- Fig. 5:: Darstellungen des Implantates (A) des teilweise geschnittenen Implantates (B) sowie des teilweise geschnittenen lmplantatkernes (C).

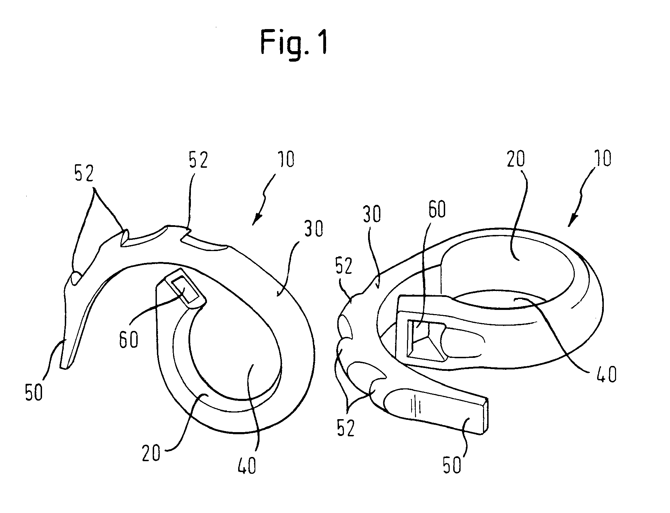

Fig. 1 zeigt in unterschiedlichen perspektivischen Darstellungen das Implantat 10. Das Implantat 10 weist einen Kontaktbereich 20 auf, mit dem das Implantat 10 im implantierten Zustand mit Körpergewebe in Verbindung steht. Das Implantat 10 gemäß Fig. 1 dient der Behandlung der gastroösophagealen Reflux-Krankheit und wird um den unteren Bereich der Speiseröhre gelegt. Der Kontaktbereich 20 steht somit mit dem äußeren Gewebe der Speiseröhre in Verbindung.

Der Kontaktbereich 20 weist eine offenporige poröse Struktur auf, deren Poren einen Durchmesser im Bereich zwischen 100 und 300 µm aufweisen. Diese Poren sind durch interkonnektive Poren miteinander verbunden. Die interkonnektiven Poren weisen einen Durchmesser vorzugsweise im Bereich zwischen 10 und 30 µm. Die Porosität ist vorzugsweise größer als 65 %. Der Kontaktbereich 20 ermöglicht es Fibroblasten des Speiseröhrengewebes in den Kontaktbereich 20, sowie auch in dahinter liegende Bereiche des Implantates einzuwachen, die vorzugsweise ebenfalls eine poröse Struktur aufweisen.

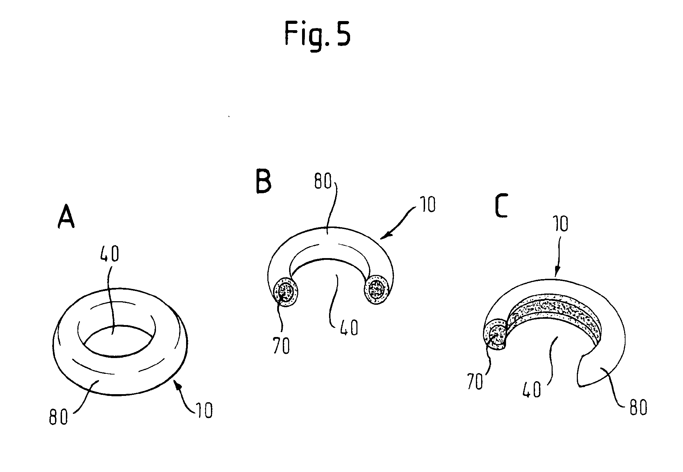

Das Implantat 10 besteht aus einem die poröse Struktur aufweisenden Kern 70 (vgl. Fig. 5), der abgesehen von dem Kontaktbereich 20 von einer Umhüllung 80 umgeben ist, die eine glatte Oberfläche 30 aufweist.

Wie aus Fig. 1 ersichtlich, umgibt ein im wesentlichen ringförmiger Abschnitt des Implantates 10 einen im wesentlichen kreisförmigen Aufnahmebereich 40, dessen Umfangsbereich durch die Kontaktfläche 20 gebildet wird. An die Kontaktfläche 20 angrenzend schließt sich die glatte Umhüllung an.

In den Endbereichen des ringförmigen Abschnittes befindet sich zum einen der steckerförmige Abschnitt 50 und zum anderen die Steckeraufnahme 60, in die der steckerförmige Abschnitt 50 einführbar ist. Wie aus Fig. 1 ersichtlich, weist der steckerförmige Abschnitt 50 mehrere Arretierungsmittel 52 auf, die gewährleisten, daß der steckerförmige Abschnitt 50 nicht oder nur mit größerem Kraftaufwand aus der Steckeraufnahme 60 entnehmbar ist. Aufgrund der mehreren Arretierungsmittel 52 kann die Position des steckerförmigen Abschnitts 50 relativ zur Steckeraufnahme 60 und somit die Größe des Aufnahmebereichs 40 verändert werden. Daraus ergibt sich der Vorteil, daß das Implantat 10 unabhängig von der Größe des zu umgebenden Gewebes für eine Vielzahl von Patienten/Anwendungen einsetzbar ist. Der Kern 70 bzw. die die Kontaktfläche 20 bildende poröse Struktur des Implantates 10 besteht vorzugsweise aus einem thermoplastischen Polyetherpolyurethan.

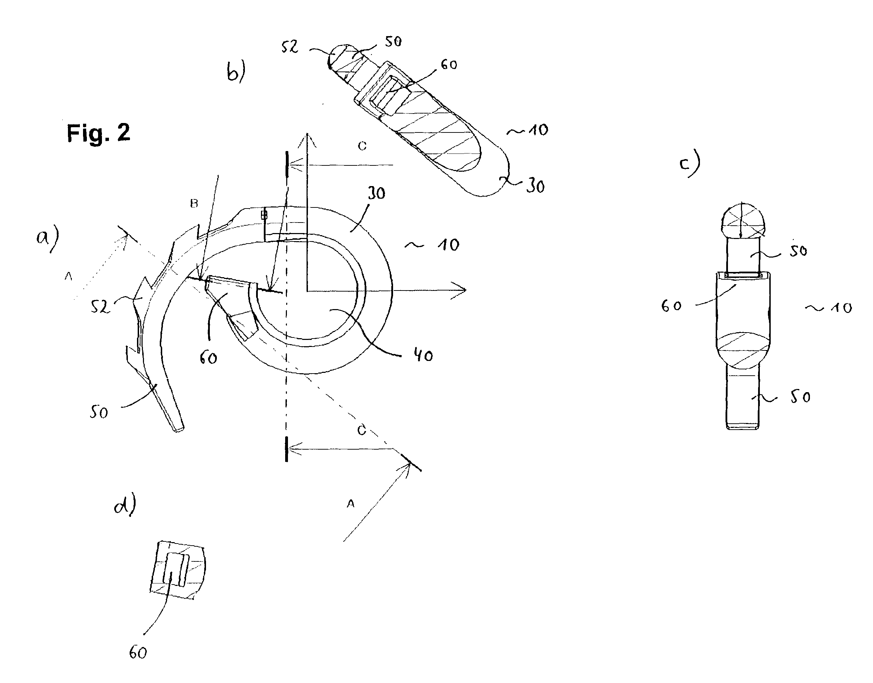

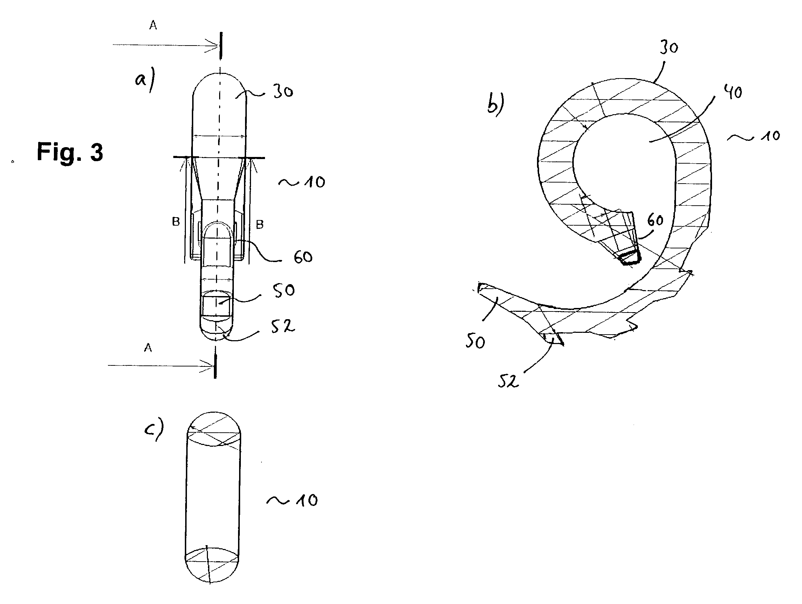

Die Figuren 2 und 3 zeigen das erfindungsgemäße Implantat in unterschiedlichen Ansichten bzw. Schnittdarstellungen. Die verwendeten Bezugszeichen entsprechen denen zu Fig. 1 und bezeichnen entsprechend identische Bauteile. Fig. 2a zeigt eine Seitenansicht des erfindungsgemäßen Implantats 10, Fig. 2b eine Schnittansicht gemäß der Linie A-A in Fig. 2a. Fig. 2c zeigt eine Schnittansicht gemäß Linie C-C in Fig. 2a und Fig. 2d zeigt eine Schnittansicht gemäß Linie B-B in Fig. 2.

Fig. 3a zeigt eine Vorderansicht des erfindungsgemäßen Implantates A. Aus Fig. 3b geht eine Schnittansicht gemäß Linie A-A gemäß Fig. 3a hervor. Fig. 3c zeigt schließlich eine Schnittansicht gemäß Linie B-B in Fig. 3a.

Die Figuren 2 und 3 zeigen nochmals eine mögliche Ausführungsform des Implantates mit einem im wesentlichen kreisringförmigen Abschnitts, der den ebenfalls im wesentlichen kreisringförmigen Aufnahmebereich begrenzt. Im einen Endbereich des kreisförmigen Bereiches des Implantates 10 findet sich die Steckeraufnahme 60 und im anderen Endbereich der steckerförmige Abschnitt 50. Die darauf auf seiner Außenseite angeordneten Arretierungsmittel 52 werden durch Vorsprünge gebildet, die sich auf der Außenseite des steckerförmigen Abschnitts 50 erstrecken und sich relativ zu dessen Oberfläche schräg erstrecken, wie dies aus Fig. 2a und Fig. 3b ersichtlich ist.

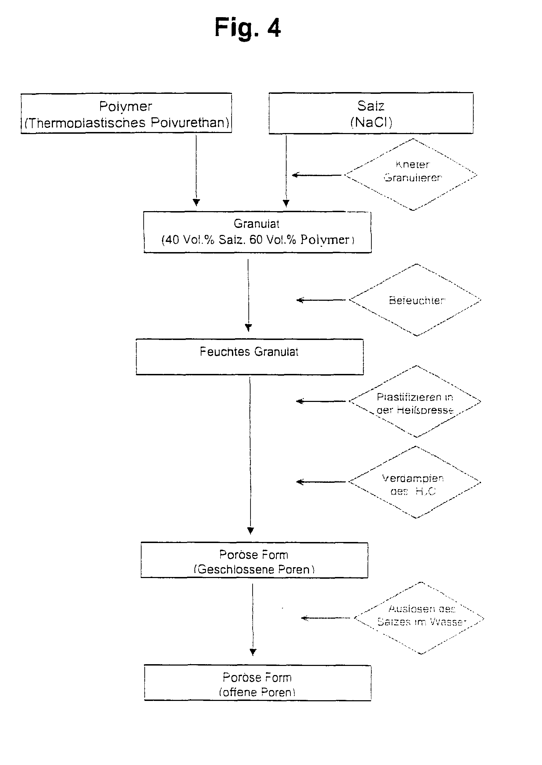

Ein Herstellprozeß für die poröse Struktur ist exemplarisch in Fig. 4 angegeben. Zunächst werden NaCl-Partikel in einer Planetenmühle zerkleinert und anschließend mit Hilfe einer Siebfraktionierung klassifiziert. Es werden Teilchen mit einer Größe von 100 bis 200 µm verwendet. Die NaCl-Partikel werden mit thermoplastischen Polyether Polyurethan TPU in einem Zweiwellenkneter 36 D extrudiert und anschließend zu Granulatkörnern zerhackt. Das Granulat besteht vorzugsweise aus ca. 40 Vol.% Salz sowie aus ca. 60 Vol.% Polymer.

Diese Granulate werden sodann der Luftfeuchtigkeit ausgesetzt, wobei Wasser absorbiert wird. Das vom Granulat absorbierte Wasser dient später als Schaummittel.

Die Granulate werden in eine Metallform eingefüllt und in einer Heißpresse bzw. einer Spritzgußmaschine plastifiziert. Verfahrensparameter bei diesem Verfahrensschritt sind Temperatur, Befüllmenge der Form, Feuchtigkeitsaufnahme des Granulates sowie die Kühl- und Heizrate der Heißpresse. Das Verfahren ist nicht auf die Verwendung einer Heißpresse beschränkt, sondern kann beispielsweise auch in einer Spritzgußmaschine angewendet werden.

Bei der Plastifizierung in der Heißpresse / Spritzgußmaschine verdampft das Wasser im Granulat, wodurch eine geschlossene Porenstruktur im Polymer entsteht. Anschließend wird das Salz mittels Wasser ausgewaschen, wobei eine offenporige Struktur erhalten wird.

Das in Fig. 4 dargestellte Verfahren arbeitet abgesehen von der Verwendung von Wasser ohne Verwendung von Lösungsmitteln. Durch Variation der Verfahrensparameter können Porengröße und Porositäten eingestellt werden, auch die Größe und Wandstärke ist veränderbar. Der in Fig. 4 dargestellte Prozeß verändert nicht die chemische Zusammensetzung des Polymers.

Fig. 5 zeigt das Implantat 10 ohne den in Fig. 1 dargestellten Verschluß und Arretierungsmechanismus. Das Implantat 10 gemäß Fig. 5 wurde mittels einer Spritzgußmaschine erhalten. Fig. 5, A zeigt die äußere solide Umhüllung 80 des Implantates. In dem geschnittenen Zustand in Fig. 5, B ist ersichtlich, daß die Umhüllung 80 einen Kern 70 ergibt, der die genannte gewünschte poröse Struktur aufweist. Umhüllung 80 und Kern 70 bestehen aus demselben Polymer, weisen jedoch aufgrund ihrer unterschiedlichen Durchströmung der Spritzgußmaschine eine unterschiedliche Struktur auf. So weist der die Umhüllung 80 bildende Bereich eine weitgehend glatte Oberfläche und kein oder wenig Salz auf, während der den Kern 70 bildende Bereich porös ist und Salz enthält. Fig. 5, C zeigt die Anordnung in Fig. 5, B nach dem Auslösen des Salzes in Wasser.

Entfernt man mechanisch den den Aufnahmebereich 40 des Implantates 10 begrenzenden Bereichs der Umhüllung 80, so daß der Kern 70 freigelegt wird, erhält man die gewünschte Struktur gemäß Fig. 1. Beim Abtrennen dieses Bereiches der Umhüllung 80 kann selbstverständlich auch der poröse Kern 70 geschnitten werden, so daß der Aufnahmebereich 70 durch eine im wesentlichen zylindrische, den Kontaktbereich 20 bildende Wandung gebildet wird.

## Patentansprüche

1. Implantat (10), vorzugsweise Implantat zur Behandlung der gastroösophagealen Refluxkrankheit, mit einem Kontaktbereich (20), mit dem das Implantat (10) im implantierten Zustand mit Körpergewebe in Verbindung steht, **dadurch gekennzeichnet, daß** der Kontaktbereich (20) wenigstens bereichsweise eine poröse Struktur aufweist.

2. Implantat (10) nach Anspruch 1, **dadurch gekennzeichnet, daß** dem Kontaktbereich (20) benachbarte Bereiche des Implantates (10) ebenfalls eine poröse Struktur aufweisen.

3. Implantat (10) nach Anspruch 1 oder 2, **dadurch gekennzeichnet, daß** die Poren der porösen Struktur miteinander in Verbindung stehen.

4. Implantat (10) nach einem der vorhergehenden Ansprüche, **dadurch gekennzeichnet, daß** die poröse Struktur offenporig ausgeführt ist.

5. Implantat (10) nach einem der vorhergehenden Ansprüche, **dadurch gekennzeichnet, daß** die Poren der porösen Struktur einen Durchmesser im Bereich zwischen 100 und 300 µm, und die Verbindungen (interkonnektive Poren) zwischen diesen Poren einen Durchmesser im Bereich zwischen 10 und 30 µm aufweisen.

6. Implantat (10) nach einem der vorhergehenden Ansprüche, **dadurch gekennzeichnet, daß** die poröse Struktur eine Porosität von mindestens 50 % und vorzugsweise von mindestens 65 % aufweist.

7. Implantat (10) nach einem der vorhergehenden Ansprüche, **dadurch gekennzeichnet, daß** die nicht den Kontaktbereich (20) bildende Oberfläche (30) des Implantates (10) glatt ausgeführt ist.

8. Implantat (10) nach einem der vorhergehenden Ansprüche, **dadurch gekennzeichnet, daß** das Implantat (10) einen Aufnahmebereich (40) zur wenigstens teilweisen Aufnahme von Körpergewebe, insbesondere eines Abschnittes der Speiseröhre aufweist.

9. Implantat (10) nach Anspruch 8, **dadurch gekennzeichnet, daß** die Größe des Aufnahmebereiches (40) veränderbar ist.

10. Implantat (10) nach Anspruch 8 oder 9, **dadurch gekennzeichnet, daß** das Implantat (10) einen im wesentlichen ringförmigen Abschnitt aufweist, der den Aufnahmebereich (40) zumindest teilweise umgibt.

11. Implantat (10) nach einem der vorhergehenden Ansprüche, **dadurch gekennzeichnet, daß** das Implantat einen steckerförmigen Abschnitt (50) und eine Stekkeraufnahme (60) aufweist, in die der steckerförmige Abschnitt (50) einführbar ist.

12. Implantat (10) nach Anspruch 11, **dadurch gekennzeichnet, daß** der steckerförmige Abschnitt (50) über ein oder mehrere Arretierungsmittel (52) verfügt, mittels derer der steckerförmige Abschnitt (50) in der Steckeraufnahme (60) arretierbar ist.

13. Implantat (10) nach Anspruch 11 oder 12, **dadurch gekennzeichnet, daß** das Implantat (10) einen den Aufnahmebereich (40) zumindest teilweise umgebenden Abschnitt aufweist, an dessen einen Endbereich der steckerförmige Abschnitt (50) und an dessen anderen Endbereich die Steckeraufnahme (60) angeordnet ist.

14. Implantat (10) nach einem der vorhergehenden Ansprüche, **dadurch gekennzeichnet, daß** das Implantat (10) einen die poröse Struktur aufweisenden Kern (70) sowie eine Umhüllung (80) aufweist, die den Kern (70) außer im Kontaktbereich (20) des Implantates (10) umgibt.

15. Implantat (10) nach einem der vorhergehenden Ansprüche, **dadurch gekennzeichnet, daß** die poröse Struktur ein oder mehrere Polymere enthält oder aus einem oder mehreren Polymeren besteht.
